# EUROPÄISCHE PATENTSCHRIFT

(11) **EP 3 004 041 B1**
(45) Veröffentlichungstag und Bekanntmachung des Hinweises auf die Patenterteilung: **06.05.2020**
(21) Anmeldenummer: 14727786.7
(22) Anmeldetag: 23.05.2014
(51) Int. Cl.: C07C 45/35, C07C 51/235, C07C 47/22, C07C 57/04, B01J 23/88, B01J 23/89

(54) **VERFAHREN ZUR HERSTELLUNG VON ACROLEIN**
METHOD FOR PRODUCING ACROLEIN
PROCÉDÉ DE PRODUCTION D'ACROLÉINE

(30) Priorität: 03.06.2013 EP 13170210; 03.06.2013 US 201361830459 P
(43) Veröffentlichungstag der Anmeldung: 13.04.2016
(73) Patentinhaber: Evonik Operations GmbH, 45128 Essen (DE)
(72) Erfinder: STEFFAN, Martin, Singapore 098652 (SG); MÜLLER, Helmut, 63637 Jossgrund (DE); ROTH, Philipp, 63456 Hanau (DE); WECKBECKER, Christoph, 63584 Gründau (DE); JAKOB, Harald, 63594 Hasselroth (DE)
(74) Vertreter: Evonik Patent Association
(86) Internationale Anmeldenummer: PCT/EP2014/060717
(87) Internationale Veröffentlichungsnummer: WO 2014/195157

(56) Entgegenhaltungen:
- EP-A2- 1 981 835
- EP-B1- 0 959 062
- WO-A1-02/00587
- US-A- 3 009 960

## Beschreibung

Die vorliegende Erfindung betrifft ein Verfahren zur Herstellung von Acrolein aus Propylen durch katalytische Gasphasenoxidation mit molekularem Sauerstoff (z.B. Luft). Die Erfindung betrifft ferner die Verwendung bestimmter Propylen-enthaltende Ausgangsmaterialien, z.B. "Refinery Grade" Propylen, zur Herstellung von Acrolein.

Acrolein wird als Zwischenprodukt in der großtechnischen Herstellung der synthetischen Aminosäure DL-Methionin und dessen Hydroxyanalogon DL-2-Hydroxy-4-methylmercaptobuttersäure eingesetzt, die erhebliche wirtschaftliche Bedeutung als Mischfutterbestandteil in der Tierernährung besitzen.

Die technische Herstellung in der chemischen Industrie erfolgt weitestgehend über die Gasphasenoxidation von Propan oder Propylen in Gegenwart geeigneter heterogener Katalysatoren. Diese Partialoxidation erfolgt mit Luft als Oxidationsmittel bei Temperaturen im Bereich von etwa 300 bis 400 °C wobei z.B. Rohrbündelreaktoren eingesetzt werden, in denen die stark exotherme Reaktion mit Salzbädern gekühlt wird. Es wird nur eine relativ verdünnte Mischung von Propylen mit Luft (meist noch in Gegenwart von Wasserdampf) eingesetzt, um die Bildung explosionsfähiger Gemische zu vermeiden. Die mit modernen Katalysatoren erzielbaren Ausbeuten an Acrolein betragen etwa 83 bis 90% bezüglich Propylen (Ullmanns Encyclopedia of Industrial Chemistry, "Acrolein and Methacrolein"), als Nebenprodukte entstehen 5 bis 10 % Acrylsäure und 3 bis 6 % Kohlenstoffmonoxid und Kohlenstoffdioxid neben nicht umgesetztem Propylen.

Neben diesen sind noch weitere Nebenkomponenten bekannt, wie zum Beispiel beschrieben in der US 6,057,481 und DT 1618889. Dies sind u.a. Acetaldehyd, Formaldehyd, organische Säuren (z.B. Essigsäure), Ketone und Wasser. Das Auftreten von Nebenkomponenten hängt dabei neben unselektiven Reaktionen des Propylens am Katalysator stark von Verunreinigungen des eingesetzten Propylens ab. Propylen ist generell in drei Qualitäten erhältlich, die sich durch den PropylenGehalt unterscheiden, der für "polymer grade" typischerweise bei über 99,1 %, für "chemical grade" im Bereich von 92 bis 96 % und für "refinery grade" zwischen 50 und 70 % liegt (Process Economics Program Report 267, "PROPYLENE PRODUCTION", October 2008). Hauptnebenkomponente dabei ist Propan, aber auch höhere gesättigte und ungesättigte Kohlenwasserstoffe sowie Schwefelverbindungen, wie in Tabelle 4.9 des zuvor genannten Berichts dargestellt.

Es wird ebenfalls beschrieben, dass "refinery grade" Propylen für gewisse chemische Prozesse direkt eingesetzt werden kann, wie z.B. bei der Herstellung von Cumol oder Isopropanol. Es wird jedoch erwähnt, dass die Konzentration an Schwefel nicht größer als etwa 2 ppm sein sollte (vgl. Tabelle 3.2 in Process Economics Program Report 267, "PROPYLENE PRODUCTION", October 2008). Dies deckt sich mit der Patentanmeldung US 2004/0192986 A1 von Wu et al., in der beschrieben wird, dass die Abtrennung von Schwefelkomponenten aus olefinischen Kohlenwasserstoffströmen zu einer Erhöhung der Standzeit und Effizienz der Katalysatoren in der Cumolsynthese führt.

In der EP 09 595 062 B1 wird beschrieben, dass gesättigte Kohlenwasserstoffe wie Propan bei der Umsetzung von Propylen zu Acrolein (und nachfolgend zu Acrylsäure) nur eine geringe Reaktivität mit dem Katalysator aufweisen und somit nur wenig Einfluss auf die Katalysatorperformance und Katalysatorstandzeit ausüben. Kritisch sei jedoch das Vorhandensein von ungesättigten Kohlenwasserstoffen im Propylenfeed, da sie unter den vorherrschenden Bedingungen mit dem Katalysator reagieren und so zu Nebenprodukten führen und die Katalysatorperformance verschlechtern können.

Überraschend wurde nun gefunden, dass man bei der Herstellung von Acrolein durch katalytische Gasphasenoxidation von Propylen mit molekularem Sauerstoff auf Propylengas niedrigerer Qualität zurückgreifen kann, ohne dabei das Leistungsverhalten des verwendeten Oxidationskatalysators im Vergleich zur Verwendung von Propylen hoher Qualität wesentlich zu verschlechtern. Erwartet wurde, dass die Ausbeute an Acrolein beim Einsatz von Schwefelkomponenten bei längerer Laufzeit zurückgeht, da Nebenreaktionen durch die Schwefelverbindung im Reaktionssystem ermöglicht werden können bzw. eine Vergiftung des Katalysators erfolgt und so die Selektivität zu Acrolein abnimmt. Insbesondere wurde gefunden, dass das eingesetzte Propylen bzw. ein Propylen enthaltendes Reaktionsgas Schwefel und/oder ungesättigte Kohlenwasserstoffe enthalten darf, ohne dass sich der Umsatz an Propylen und die Ausbeute an Acrolein wesentlich verschlechtert im Vergleich zur Verwendung von Propylen ohne die genannten Verunreinigungen.

Darüber hinaus wurde "refinery grade"-Propylen (75,48 Gew.-% Propylen, 24,04 Gew.-% Propan, 0,28 Gew.-% Ethan, Rest gesättigte und ungesättigte C2- bis C5-Kohlenwasserstoffe (ausgenommen C₃ und Ethan), 0,7 mg/kg Schwefel, Wassergehalt unter 20 mg/kg) als Feedgas für die Partialoxidation in Gegenwart von molekularen Sauerstoff an einem Mischoxidkatalysator, wie in DE 10 2006 015710 A1 beschrieben, eingesetzt. Es konnte analog zu den Versuchen mit Schwefelkomponente kein signifikanter Einfluss auf die Katalysatorleistung festgestellt werden.

Das im "refinery grade"-Propylen enthaltene Propan kann außerdem in vorteilhafter Weise einerseits als Inertgas (unter den weiter oben beschriebenen Bedingungen) und andererseits als Brenngas in einer thermischen Nachverbrennung, die dem Prozess nachgeschaltet ist, eingesetzt werden, wodurch sich wiederum Methan einsparen lässt, welches üblicherweise als Brenngas dient.

Gegenstand der vorliegenden Erfindung ist nun ein Verfahren zur Herstellung von Acrolein durch katalytische Gasphasenoxidation, wobei das Verfahren folgende Schritte umfasst:
a) Bereitstellen eines Propylen und molekularen Sauerstoff enthaltenden Reaktionsgases, und
b) Inkontaktbringen des Reaktionsgases mit einem Oxidationskatalysator unter Bildung eines Acrolein enthaltenden Gasgemisches, wobei der Oxidationskatalysator ein Mischoxidkatalysator ist, der ein oder mehrere Basiskomponenten ausgewählt aus Molybdän, Vanadium und Wolfram enthält oder daraus besteht,
dadurch gekennzeichnet, dass im bereitgestellten Reaktionsgas gleich oder mehr als 125 Gew.-ppm Schwefel in Form beliebiger Schwefelkomponenten enthalten ist, wobei in jedem Fall im bereitgestellten Reaktionsgas nicht mehr als 5000 Gew.-ppm Schwefel in Form beliebiger Schwefelkomponenten und nicht mehr als 5000 Gew.-ppm an ungesättigten Kohlenwasserstoffen ausgesucht aus der Gruppe bestehend aus C₂H₄, C₃H₄, C₄H₈, C₄H₆, C₅H₁₀, C₅H₈ und Mischungen davon enthalten ist.

Der Gehalt an Schwefel (in Form beliebiger Schwefelkomponenten) im bereitgestellten Reaktionsgas wird bestimmt durch die Norm EN 24 260 (Verbrennung nach Wickbold). Der Gehalt an ungesättigten Kohlenwasserstoffen ausgesucht aus der Gruppe bestehend aus C₂H₄, C₃H₄, C₄H₈, C₄H₆, C₅H₁₀, C₅H₈ und Mischungen davon im bereitgestellten Reaktionsgas wird bestimmt durch die Norm DIN 51619 (Gaschromotagraphie). Sind alternative Varianten der Durchführungsvorschriften in den Normen genannt, müssen die oben angegebenen Gew.-ppm-Werte nach wenigsten einer der möglichen alternativen Varianten erreicht werden.

Das erfindungsgemäße Verfahren erlaubt auch, dass im bereitgestellten Reaktionsgas zusätzlich mehr als 50 Gew.-ppm an ungesättigten Kohlenwasserstoffen, ausgesucht aus der Gruppe bestehend aus C₂H₄, C₃H₄, C₄H₈, C₄H₆, C₅H₁₀, C₅H₈ und Mischungen davon, enthalten sein können.

Mit ungesättigten Kohlenwasserstoffen, ausgesucht aus der Gruppe bestehend aus C₂H₄, C₃H₄, C₄H₈, C₄H₆, C₅H₁₀, C₅H₈ und Mischungen davon, sind insbesondere gemeint Ethen, Propadien, Methylacetylen, Isobuten, 1-Buten, cis-2-Buten, trans-2-Buten, Butadien, 1-Penten und Mischungen davon.

Nach dem erfindungsgemäßen Verfahren kann der Gesamtgehalt an Schwefel in Form beliebiger Schwefelkomponenten im bereitgestellten Reaktionsgas auch mehr als 125 Gew.-ppm betragen. Bevorzugt ist es jedoch, wenn der Gesamtgehalt an Schwefel nicht mehr als 1000 Gew.-ppm beträgt.

Das erfindungsgemäße Verfahren erlaubt insbesondere, dass der Gesamtgehalt an Schwefel in Form von H₂S und/oder SO₂ im bereitgestellten Reaktionsgas mehr als 125 Gew.-ppm betragen kann, sofern der Gesamtgehalt an Schwefel in Form beliebiger Schwefelkomponenten (einschließlich H₂S und/oder SO₂) im Ausgangsmaterial bevorzugt nicht mehr als 1000 Gew.-ppm, bevorzugter nicht mehr als 500 Gew.-ppm, und besonders bevorzugt nicht mehr als 250 Gew.-ppm, beträgt.

Das erfindungsgemäße Verfahren erlaubt insbesondere auch, dass der Gesamtgehalt an ungesättigten Kohlenwasserstoffen, ausgesucht aus der Gruppe bestehend aus C₂H₄, C₃H₄, C₄H₈, C₄H₆, C₅H₁₀, C₅H₈ und Mischungen davon, im bereitgestellten Reaktionsgas mehr als 100 Gew.-ppm oder auch mehr als 150 Gew.-ppm oder mehr als 200 Gew.-ppm jedoch nicht mehr als 1500 Gew.-ppm, bevorzugt nicht mehr als 1300 Gew.-ppm, und bevorzugter nicht mehr als 1000 Gew.-ppm, betragen kann.

In einer bevorzugten Ausführungsform des erfindungsgemäßen Verfahrens ist vorgesehen, dass der Mischoxidkatalysator zusätzlich ein oder mehrere Zusatzkomponenten ausgewählt aus Wismut, Antimon, Tellur, Zinn, Eisen, Cobalt, Nickel und Kupfer enthält.

Besonders geeignete Oxidationskatalysatoren im Rahmen der vorliegenden Erfindung sind ausgewählt aus Mischoxidkatalysatoren der allgemeinen Formel (I) oder Mischungen verschiedener Mischoxidkatalysatoren der allgemeinen Formel (I), wobei gilt:

(Mo₁₂BiₐC_{b}(Co+Ni)_{c}D_{d}EₑF_{f}G_{g}Hₕ)Oₓ (I)

C = Eisen,
D = mindestens eines der Elemente aus W, P,
E = mindestens eines der Elemente aus Li, K, Na, Rb, Cs, Mg, Ca, Ba, Sr,
F = mindestens eines der Elemente aus Ce, Mn, Cr, V,
G = mindestens eines der Elemente aus Nb, Se, Te, Sm, Gd, La, Y, Pd, Pt, Ru, Ag, Au,
H = mindestens eines der Elemente aus Si, Al, Ti, Zr,
a = 0 bis 5,0,
b = 0,5 bis 5,0,
c = 2 bis 15,
d = 0,01 bis 5,0,
e = 0,001 bis 2,
f = 0,001 bis 5,
g = 0 bis 1,5,
h = 0 bis 800,
x = Zahl, die von der Wertigkeit und Häufigkeit der von Sauerstoff verschiedenen Elemente bestimmt wird.

Wie bereits ausgeführt, ist ein Vorteil des erfindungsgemäßen Verfahrens, dass man bei der Herstellung von Acrolein durch katalytische Gasphasenoxidation von Propylen auf Propylengas niedrigerer Qualität zurückgreifen kann, ohne dabei das Leistungsverhalten des verwendeten Oxidationskatalysators im Vergleich zur Verwendung von Propylen hoher Qualität wesentlich zu verschlechtern. Dies gilt wie oben beschrieben insbesondere für Verunreinigungen durch Schwefelkomponenten und/oder ungesättigte Kohlenwasserstoffe (C₂-C₅), ausgenommen C₃.

Das erfindungsgemäße Verfahren erlaubt aber auch, dass das bereitgestellte Reaktionsgas hergestellt wird durch Mischen von wenigstens "Refinery Grade"-Propylen und Luft, wobei das "Refinery Grade"-Propylen dadurch gekennzeichnet ist, dass es 0,05 bis 2,0 Gew.-%, bevorzugt 0,1 bis 1,0 Gew.-%, weiter bevorzugt 0,2 bis 0,5 Gew.-%, Ethan und/oder 9 bis 40 Gew.-%, bevorzugt 10 bis 35 Gew.-%, weiter bevorzugt 12 bis 30 Gew.-%, besonders bevorzugt 15 bis 25 Gew.-%, Propan enthält.

Das erfindungsgemäße Verfahren wird bevorzugt so durchgeführt, dass in Schritt b) das Reaktionsgas durch ein Reaktorrohr oder parallel durch mehrere gebündelte Reaktorrohre durchgeleitet wird, wobei das eine bzw. die mehreren Reaktorrohre jeweils auf einer Länge von mindestens 2 Meter, bevorzugt mindestens 2,5 Meter, besonders bevorzugt mindestens 3 Meter, mit dem Oxidationskatalysator gefüllt sind. Typischerweise sind die Reaktorrohre jeweils auf einer Länge von 1,5 bis 5 Metern, bevorzugt 2 bis 4,5 Metern, besonders bevorzugt 2,5 bis 4 Metern, mit Oxidationskatalysator beschickt. Der Innendurchmesser jedes Reaktorrohres liegt dabei im Bereich von 1,0 bis 3,5 cm, bevorzugt im Bereich von 1,5 bis 3,3 cm, besonders bevorzugt im Bereich von 2,0 bis 3,0 cm.

Weiterhin wird das erfindungsgemäße Verfahren bevorzugt so durchgeführt, dass in Schritt b) das Reaktionsgas bei einer Temperatur im Bereich von 300 bis 500 °C, bevorzugt im Bereich von 350 bis 450 °C, besonders bevorzugt im Bereich von 370 bis 430 °C, durch das Reaktorrohr oder die mehreren gebündelten Reaktorrohre durchgeleitet wird. Die Temperatur kann durch Temperieren des/der einen oder der mehreren Reaktorrohre(s) eingestellt werden, zum Beispiel durch Umgeben des/der einen oder der mehreren Reaktorrohre(s) mit einem temperierten Wärmeträgermedium oder durch direktes Aufheizen des/der einen oder der mehreren Reaktorrohre(s) mit elektrischen Heizelementen.

Es hat sich gezeigt, dass Schritt b) des erfindungsgemäßen Verfahrens über einen Zeitraum von 30 Minuten bis 2000 Stunden, bevorzugt im Bereich von 1 bis 1000 Stunden, besonders bevorzugt im Bereich von 2 bis 500 Stunden, betrieben werden kann ohne dass eine wesentliche Verschlechterung des Leistungsverhaltens des verwendeten Oxidationskatalysators eintritt; wobei nur die Zeiten addiert werden, in denen das Reaktionsgas die erfindungsgemäß angegebenen Mengen an Schwefelkomponenten und/oder ungesättigten Kohlenwasserstoffen tatsächlich enthält.

Ein weiterer Gegenstand der vorliegenden Erfindung ist die Verwendung eines Reaktionsgases zur Herstellung von Acrolein, dadurch gekennzeichnet, dass das Reaktionsgas gleich oder mehr als 125 Gew.-ppm Schwefel in Form beliebiger Schwefelkomponenten enthält, wobei in jedem Fall das Reaktionsgas nicht mehr als 5000 Gew.-ppm, bevorzugt nicht mehr als 1000 Gew.-ppm, Schwefel in Form beliebiger Schwefelkomponenten und nicht mehr als 5000 Gew.-ppm, bevorzugt nicht mehr als 1500 Gew.-ppm, an ungesättigten Kohlenwasserstoffen, ausgesucht aus der Gruppe bestehend aus C₂H₄, C₃H₄, C₄H₈, C₄H₆, C₅H₁₀, C₅H₈ und Mischungen davon, enthält.

Eine erfindungsgemäße Verwendung erlaubt auch, dass im bereitgestellten Reaktionsgas mehr als 50 Gew.-ppm an ungesättigten Kohlenwasserstoffen, ausgesucht aus der Gruppe bestehend aus C₂H₄, C₃H₄, C₄H₈, C₄H₆, C₅H₁₀, C₅H₈ und Mischungen davon, enthalten sein können.

Der Gesamtgehalt an Schwefel in Form beliebiger Schwefelkomponenten und/oder an ungesättigten Kohlenwasserstoffen, ausgesucht aus der Gruppe bestehend aus C₂H₄, C₃H₄, C₄H₈, C₄H₆, C₅H₁₀, C₅H₈ und Mischungen davon, im Reaktionsgas kann auch jeweils mehr als 20 Gew.-ppm betragen. Die erfindungsgemäße Verwendung erlaubt insbesondere, dass der Gesamtgehalt an Schwefel in Form von H₂S und/oder SO₂ im Reaktionsgas mehr als 10 Gew.-ppm oder auch mehr als 20 Gew.-ppm betragen kann sofern der Gesamtgehalt an Schwefel in Form beliebiger Schwefelkomponenten (einschließlich H₂S und/oder SO₂) im Reaktionsgas nicht mehr als 5000 Gew.-ppm, bevorzugt nicht mehr als 1000 Gew.-ppm beträgt.

Ein Gegenstand der Beschreibung ist auch die Verwendung eines Acrolein enthaltenden Gasgemisches, welches nach dem oben beschriebenen Verfahren herstellbar ist, zur Herstellung von Acrylsäure.

In einer bevorzugten Ausführungsform der zusätzlich beschriebenen Verwendung umfasst die Herstellung der Acrylsäure das Mischen des Acrolein enthaltenden Gasgemisches mit weiterem molekularen Sauerstoff und das Inkontaktbringen des Gemisches mit einem Oxidationskatalysator unter Bildung eines Acrylsäure enthaltenden Gasgemisches. Dies kann erfolgen ohne eine aufreinigende Entfernung des gegebenenfalls enthaltenen Schwefels in Form beliebiger Schwefelkomponenten und der gegebenenfalls enthaltenen ungesättigten Kohlenwasserstoffen, ausgesucht aus der Gruppe bestehend aus C₂H₄, C₄H₈, C₃H₄, C₄H₆, C₅H₁₀, C₅H₈ und Mischungen davon.

### Beispiele

### Beispiel 1

In einem Rohrreaktor mit 20 mL Katalysatorschüttung wurde die heterogenkatalysierte Partialoxidation von Propylen in Gegenwart von molekularem Sauerstoff an einem Mischmetalloxid-Kontakt, dessen Herstellvorschrift sich in DE 10 2006 015710 A1 (Beispiel 1) findet, durchgeführt. Neben Propylen und Luft wurde Stickstoff (und ggf. Dampf) als zusätzliches Inertgas eingespeist. Der Reaktor wurde durch einen elektrischen Ofen auf ca. 360°C geheizt. Die Temperatur in der Katalysatorschüttung wurde über Thermoelemente verfolgt, wobei die maximale Temperatur bei etwa 400°C lag. Die Analyse des Prozessgases wurde mittels Gaschromatographie durchgeführt.

Die Reaktion wurde zunächst unter Standardbedingungen (= Feedgas bestehend aus Propylen, Sauerstoff und Inertgas, z. B. Stickstoff) angefahren. Um den Einfluss von Schwefelkomponenten zu untersuchen, wurde über einen zusätzlichen Massendurchflussmesser eine Mischung von Schwefelkomponente in Inertgas (z. B. H₂S in Stickstoff) zudosiert und dabei parallel schrittweise das Inertgas der Standardbedingungen ersetzt, sodass der Gesamtvolumenstrom konstant gehalten wurde. Im Folgenden sind Ergebnisse nach verschiedenen Katalysatorlaufzeiten sowie der jeweilige Massenanteil an Schwefelkomponente (bezogen auf den Gesamtgasstrom) angegeben:

**Tabelle 1: Katalysatorperformance nach verschiedenen Laufzeiten mit und ohne Dosierung von H₂S.**

| **Laufzeit** | **Massenanteil H₂S** [34 g/mol] | **Massenanteil Schwefel** [32 g/mol] | **Dauer H₂S-Dosierung** | **Umsatz Propylen** | **Ausbeute Acrolein** |
|---|---|---|---|---|---|
| **[h]** | **[Gew.-ppm]** | **[Gew.-ppm]** | **[h]** | **[%]** | **[%]** |
| 50 | 0 | 0 | - | 90,1 | 80,0 |
| 135 | 0 | 0 | - | 90,6 | 80,0 |
| 315 | 220 | 207 | 150 | 91,6 | 80,1 |
| 550 | 435 | 409 | 337 | 89,9 | 80,0 |
| 810 | 870 | 819 | 260 | 90,0 | 80,6 |
| 1080 | 1500 | 1412 | 103 | 91,3 | 80,9 |

### Beispiel 2

In einen Rohrreaktor von 3400 mm Länge mit 21,7 mm Innendurchmesser wurde eine Schüttung von 2850 mm Katalysator eingebracht. Der Reaktor wird von einem Wärmeträgermedium umgeben und so bei einer gleichmäßigen Temperatur von 350°C gehalten. Der Reaktor wurde mit einem Reaktionsgas aus Propylen (9,3 Gew.%), Luft (58,3 Gew.%), Rest Inertgas beschickt. Über eine separate Regelstrecke wurde zunächst SO₂ (als Reinsubstanz und/oder als Mischung mit Inertgas) zudosiert. Der Katalysator wurde eingefahren und erst nach mehreren Tagen stationärem Betrieb wurde mit der Dosierung der Schwefelkomponente begonnen. Der Massenanteil wurde dann sukzessive erhöht.

**Tabelle 2: Katalysatorperformance nach verschiedenen Laufzeiten mit und ohne Dosierung von SO₂ bzw. H₂S.**

| **Laufzeit** | **Massenanteil SO₂** [64 g/mol] | **Massenanteil Schwefel** [32 g/mol] | **Dauer SO₂**-**Dosierung** | **Umsatz Propylen** | **Ausbeute Acrolein** |
|---|---|---|---|---|---|
| **[h]** | **[Gew.-ppm]** | **[Gew.-ppm]** | **[h]** | **[%]** | **[%]** |
| 50 | 0 | 0 | - | 97,6 | 85,6 |
| 150 | 0 | 0 | - | 97,5 | 85,6 |
| 350 | 0 | 0 | - | 97,6 | 85,4 |
| 660 | 250 | 125 | 288 | 97,4 | 85,2 |
| 1000 | 1000 | 500 | 72 | 98,3 | 85,9 |
| 1200 | 2000 | 1000 | 120 | 98,7 | 85,9 |
| 1440 | 5000 | 2500 | 72 | 97,2 | 85,3 |
| 1670 | 10000 | 5000 | 120 | 97,5 | 79,8 |

| **Regeneration** | | | | | |
|---|---|---|---|---|---|
| **Laufzeit** | **Massenanteil SO₂** | **Massenanteil Schwefel** | **Dauer SO₂**-**Dosierung** | **Umsatz Propylen** | **Ausbeute Acrolein** |
| **[h]** | **[Gew.-ppm]** | **[Gew.-ppm]** | **[h]** | **[%]** | **[%]** |
| 1800 | 500 | 250 | 72 | 97,5 | 85,2 |
| 1950 | 1000 | 500 | 72 | 97,6 | 84,7 |

Die Ergebnisse (Umsatz, Ausbeute) bis zu einer Katalysatorlaufzeit von 350 h, in der keine Schwefelkomponente dosiert wurde, zeigen die Performance des Katalysators ohne Einwirkung von Schwefelkomponenten.

Die in den Tabellen 1 und 2 angegebenen Laufzeiten sind Bruttolaufzeiten. Die jeweils angegebene Dauer der H₂S- bzw. SO₂-Dosierung mit einem bestimmten Massenanteil an H₂S bzw. SO₂ hat dabei stattgefunden in dem Zeitraum zwischen den Laufzeiten der H₂S- bzw. SO₂-Dosierung mit dem nächsthöheren bzw. nächstniedrigeren H₂S- bzw. SO₂-Massenanteil.

Beispiel: Die Dauer der SO₂-Dosierung von 288h bei 250 Gew.-ppm SO₂-Massenanteil lag in dem Intervall zwischen 350 und 1000h Laufzeit. In der übrigen Zeit des Intervalls wurde Reaktionsgas ohne SO₂-Dosierung durch den Rohrreaktor geleitet.

## Patentansprüche

1. Verfahren zur Herstellung von Acrolein durch katalytische Gasphasenoxidation, wobei das Verfahren folgende Schritte umfasst:
a) Bereitstellen eines Propylen und molekularen Sauerstoff enthaltenden Reaktionsgases, und
b) Inkontaktbringen des Reaktionsgases mit einem Oxidationskatalysator unter Bildung eines Acrolein enthaltenden Gasgemisches, wobei der Oxidationskatalysator ein Mischoxidkatalysator ist, der ein oder mehrere Basiskomponenten ausgewählt aus Molybdän, Vanadium und Wolfram enthält oder daraus besteht,
**dadurch gekennzeichnet, dass** im bereitgestellten Reaktionsgas gleich oder mehr als 125 Gew.-ppm Schwefel in Form beliebiger Schwefelkomponenten enthalten ist, wobei in jedem Fall im bereitgestellten Reaktionsgas nicht mehr als 5000 Gew.-ppm Schwefel in Form beliebiger Schwefelkomponenten und nicht mehr als 5000 Gew.-ppm an ungesättigten Kohlenwasserstoffen, ausgesucht aus der Gruppe bestehend aus C₂H₄, C₃H₄, C₄H₈, C₄H₆, C₅H₁₀, C₅H₈ und Mischungen davon, enthalten ist.

2. Verfahren nach Anspruch 1, **dadurch gekennzeichnet, dass** im bereitgestellten Reaktionsgas mehr als 50 Gew.-ppm an ungesättigten Kohlenwasserstoffen, ausgesucht aus der Gruppe bestehend aus C₂H₄, C₄H₈, C₃H₄, C₄H₆, C₅H₁₀, C₅H₈ und Mischungen davon enthalten ist.

3. Verfahren nach einem der vorherigen Ansprüche, **dadurch gekennzeichnet, dass** im bereitgestellten Reaktionsgas mehr als 125 Gew.-ppm jedoch nicht mehr als 1000 Gew.-ppm Schwefel in Form beliebiger Schwefelkomponenten enthalten ist.

4. Verfahren nach einem der vorherigen Ansprüche, **dadurch gekennzeichnet, dass** im bereitgestellten Reaktionsgas mehr als 100 Gew.-ppm jedoch nicht mehr als 1500 Gew.-ppm an ungesättigten Kohlenwasserstoffen, ausgesucht aus der Gruppe bestehend aus C₂H₄, C₃H₄, C₄H₈, C₄H₆, C₅H₁₀, C₅H₈ und Mischungen davon enthalten ist.

5. Verfahren nach einem der vorherigen Ansprüche, **dadurch gekennzeichnet, dass** im bereitgestellten Reaktionsgas mehr als 150 Gew.-ppm, jedoch nicht mehr als 1300 Gew.-ppm an ungesättigten Kohlenwasserstoffen ausgesucht aus der Gruppe bestehend aus C₂H₄, C₃H₄, C₄H₈, C₄H₆, C₅H₁₀, C₅H₈ und Mischungen davon enthalten ist.

6. Verfahren nach einem der vorherigen Ansprüche, **dadurch gekennzeichnet, dass** der Mischoxidkatalysator zusätzlich ein oder mehrere Zusatzkomponenten ausgewählt aus Wismut, Antimon, Tellur, Zinn, Eisen, Cobalt, Nickel und Kupfer enthält.

7. Verfahren nach Anspruch 76, **dadurch gekennzeichnet, dass** der Oxidationskatalysator ein Mischoxidkatalysator der allgemeinen Formel (I) oder eine Mischung verschiedener Mischoxidkatalysatoren der allgemeinen Formel (I) ist, wobei gilt:
(Mo₁₂BiₐC_{b}(Co+Ni)_{c}D_{d}EₑF_{f}G_{g}Hₕ)Oₓ (I)
C = Eisen,
D = mindestens eines der Elemente aus W, P,
E = mindestens eines der Elemente aus Li, K, Na, Rb, Cs, Mg, Ca, Ba, Sr,
F = mindestens eines der Elemente aus Ce, Mn, Cr, V,
G = mindestens eines der Elemente aus Nb, Se, Te, Sm, Gd, La, Y, Pd, Pt, Ru, Ag, Au,
H = mindestens eines der Elemente aus Si, Al, Ti, Zr,
a = 0 bis 5,0,
b = 0,5 bis 5,0,
c = 2 bis 15,
d = 0,01 bis 5,0,
e = 0,001 bis 2,
f = 0,001 bis 5,
g = 0 bis 1,5,
h = 0 bis 800,
x = Zahl, die von der Wertigkeit und Häufigkeit der von Sauerstoff verschiedenen Elemente bestimmt wird.

8. Verfahren nach einem der vorherigen Ansprüche, wobei das bereitgestellte Reaktionsgas hergestellt wird durch Mischen von wenigstens "Refinery Grade"-Propylen und Luft, wobei das "Refinery Grade"-Propylen **dadurch gekennzeichnet ist, dass** es 0,05 bis 2,0 Gew.-% Ethan und/oder 9 bis 40 Gew.-% Propan enthält.

9. Verwendung eines Reaktionsgases zur Herstellung von Acrolein, **dadurch gekennzeichnet, dass** das Reaktionsgas gleich oder mehr als 125 Gew.-ppm Schwefel in Form beliebiger Schwefelkomponenten enthält, wobei in jedem Fall das Reaktionsgas nicht mehr als 5000 Gew.-ppm Schwefel in Form beliebiger Schwefelkomponenten und nicht mehr als 5000 Gew.-ppm an ungesättigten Kohlenwasserstoffen, ausgesucht aus der Gruppe bestehend aus C₂H₄, C₃H₄, C₄H₈, C₄H₆, C₅H₁₀, C₅H₈ und Mischungen davon, enthält.

10. Verwendung nach Anspruch 9, wobei das Reaktionsgas mehr als 50 Gew.-ppm an ungesättigten Kohlenwasserstoffen, ausgesucht aus der Gruppe bestehend aus C₂H₄, C₃H₄, C₄H₈, C₄H₆, C₅H₁₀, C₅H₈ und Mischungen davon, enthält.

## Claims

1. Process for preparing acrolein by catalytic gas phase oxidation, said process comprising the following steps:
a) providing a reaction gas comprising propylene and molecular oxygen, and
b) contacting the reaction gas with an oxidation catalyst to form a gas mixture comprising acrolein, wherein the oxidation catalyst is a mixed oxide catalyst comprising or consisting of one or more base components selected from molybdenum, vanadium and tungsten,
**characterized in that** not less than 125 ppm by weight of sulphur in the form of any sulphur components is present in the reaction gas provided, but not more than 5000 ppm by weight of sulphur in the form of any sulphur components and not more than 5000 ppm by weight of unsaturated hydrocarbons selected from the group consisting of C₂H₄, C₃H₄, C₄H₈, C₄H₆, C₅H₁₀, C₅H₈ and mixtures thereof is present in each case in the reaction gas provided.

2. Process according to Claim 1, **characterized in that** more than 50 ppm by weight of unsaturated hydrocarbons selected from the group consisting of C₂H₄, C₄H₈, C₃H₄, C₄H₆, C₅H₁₀, C₅H₈ and mixtures thereof is present in the reaction gas provided.

3. Process according to either of the preceding claims, **characterized in that** more than 125 ppm by weight but not more than 1000 ppm by weight of sulphur in the form of any sulphur components is present in the reaction gas provided.

4. Process according to any of the preceding claims, **characterized in that** more than 100 ppm by weight but not more than 1500 ppm by weight of unsaturated hydrocarbons selected from the group consisting of C₂H₄, C₃H₄, C₄H₈, C₄H₆, C₅H₁₀, C₅H₈ and mixtures thereof is present in the reaction gas provided.

5. Process according to any of the preceding claims, **characterized in that** more than 150 ppm by weight but not more than 1300 ppm by weight of unsaturated hydrocarbons selected from the group consisting of C₂H₄, C₃H₄, C₄H₈, C₄H₆, C₅H₁₀, C₅H₈ and mixtures thereof is present in the reaction gas provided.

6. Process according to any of the preceding claims, **characterized in that** the mixed oxide catalyst additionally contains one or more additional components selected from bismuth, antimony, tellurium, tin, iron, cobalt, nickel and copper.

7. Process according to Claim 6, **characterized in that** the oxidation catalyst is a mixed oxide catalyst of the general formula (I) or a mixture of various mixed oxide catalysts of the general formula (I) where:
(Mo₁₂BiₐC_{b}(Co+Ni)_{c}D_{d}EₑF_{f}G_{g}Hₕ)Oₓ (I)
C = iron,
D = at least one of the elements from W, P,
E = at least one of the elements from Li, K, Na, Rb, Cs, Mg, Ca, Ba, Sr,
F = at least one of the elements from Ce, Mn, Cr, V,
G = at least one of the elements from Nb, Se, Te, Sm, Gd, La, Y, Pd, Pt, Ru, Ag, Au,
H = at least one of the elements from Si, Al, Ti, Zr,
a = 0 to 5.0,
b = 0.5 to 5.0,
c = 2 to 15,
d = 0.01 to 5.0,
e = 0.001 to 2,
f = 0.001 to 5,
g = 0 to 1.5,
h = 0 to 800,
x = number which is determined by the valency and frequency of the elements other than oxygen.

8. Process according to any of the preceding claims, wherein the reaction gas provided is produced by mixing at least refinery grade propylene and air, the refinery grade propylene being **characterized in that** it contains 0.05 to 2.0% by weight of ethane and/or 9 to 40% by weight of propane.

9. Use of a reaction gas for preparation of acrolein, **characterized in that** the reaction gas contains not less than 125 ppm by weight of sulphur in the form of any sulphur components the reaction gas in each case containing not more than 5000 ppm by weight of sulphur in the form of any sulphur components and not more than 5000 ppm by weight of unsaturated hydrocarbons, selected from the group consisting of C₂H₄, C₃H₄, C₄H₈, C₄H₆, C₅H₁₀, C₅H₈ and mixtures thereof.

10. Use according to Claim 9, wherein the reaction gas contains more than 50 ppm by weight of unsaturated hydrocarbons selected from the group consisting of C₂H₄, C₃H₄, C₄H₈, C₄H₆, C₅H₁₀, C₅H₈ and mixtures thereof.

## Revendications

1. Procédé pour la préparation d'acroléine par oxydation catalytique en phase gazeuse, le procédé comprenant les étapes suivantes :
a) mise à disposition d'un gaz de réaction contenant du propylène et de l'oxygène moléculaire, et
b) mise en contact du gaz de réaction avec un catalyseur d'oxydation avec formation d'un mélange de gaz contenant de l'acroléine, le catalyseur d'oxydation étant un catalyseur de type oxyde mixte, qui contient ou qui est constitué d'un ou plusieurs composants de base choisis parmi le molybdène, le vanadium et le tungstène,
**caractérisé en ce que** dans le gaz de réaction mis à disposition, 125 ppm en poids ou plus de soufre sous forme de quelconques composants à base de soufre sont contenus, en chaque cas pas plus de 5 000 ppm en poids de soufre sous forme de quelconques composants à base de soufre et pas plus de 5 000 ppm en poids d'hydrocarbures insaturés, choisis dans le groupe constitué par C₂H₄, C₃H₄, C₄H₈, C₄H₆, C₅H₁₀, C₅H₈ et des mélanges correspondants, étant contenus dans le gaz de réaction mis à disposition.

2. Procédé selon la revendication 1, **caractérisé en ce que** dans le gaz de réaction mis à disposition, plus de 50 ppm en poids d'hydrocarbures insaturés, choisis dans le groupe constitué par C₂H₄, C₄H₈, C₃H₄, C₄H₆, C₅H₁₀, C₅H₈ et des mélanges correspondants, sont contenus.

3. Procédé selon l'une quelconque des revendications précédentes, **caractérisé en ce que** dans le gaz de réaction mis à disposition, plus de 125 ppm en poids mais pas plus de 1 000 ppm en poids de soufre sous forme de quelconques composants à base de soufre sont contenus.

4. Procédé selon l'une quelconque des revendications précédentes, **caractérisé en ce que** dans le gaz de réaction mis à disposition, plus de 100 ppm en poids mais pas plus de 1 500 ppm en poids d'hydrocarbures insaturés, choisis dans le groupe constitué par C₂H₄, C₃H₄, C₄H₈, C₄H₆, C₅H₁₀, C₅H₈ et des mélanges correspondants, sont contenus.

5. Procédé selon l'une quelconque des revendications précédentes, **caractérisé en ce que** dans le gaz de réaction mis à disposition, plus de 150 ppm en poids mais pas plus de 1 300 ppm en poids d'hydrocarbures insaturés, choisis dans le groupe constitué par C₂H₄, C₃H₄, C₄H₈, C₄H₆, C₅H₁₀, C₅H₈ et des mélanges correspondants, sont contenus.

6. Procédé selon l'une quelconque des revendications précédentes, **caractérisé en ce que** le catalyseur de type oxyde mixte contient de plus un ou plusieurs composants supplémentaires choisis dans le groupe du bismuth, de l'antimoine, du tellure, de l'étain, du fer, du cobalt, du nickel et du cuivre.

7. Procédé selon la revendication 6, **caractérisé en ce que** le catalyseur d'oxydation est un catalyseur de type oxyde mixte de formule générale (I) ou un mélange de différents catalyseurs de type oxyde mixte de formule générale (I), où :
(Mo₁₂BiₐC_{b}(Co+Ni)_{c}D_{d}EₑF_{f}G_{g}Hₕ)Oₓ (I)
C = fer,
D = au moins un des éléments parmi W, P,
E = au moins un des éléments parmi Li, K, Na, Rb, Cs, Mg, Ca, Ba, Sr,
F = au moins un des éléments parmi Ce, Mn, Cr, V,
G = au moins un des éléments parmi Nb, Se, Te, Sm, Gd, La, Y, Pd, Pt, Ru, Ag, Au,
H = au moins un des éléments parmi Si, Al, Ti, Zr,
a = 0 à 5,0,
b = 0,5 à 5,0,
c = 2 à 15,
d = 0,01 à 5,0,
e = 0,001 à 2,
f = 0,001 à 5,
g = 0 à 1,5,
h = 0 à 800,
x = un nombre qui est déterminé par la valence et l'abondance des éléments différents de l'oxygène.

8. Procédé selon l'une quelconque des revendications précédentes, le gaz de réaction mis à disposition étant préparé par mélange d'au moins un propylène de « qualité de raffinerie » et d'air, le propylène de « qualité de raffinerie » étant **caractérisé en ce qu'**il contient 0,05 à 2,0 % en poids d'éthane et/ou 9 à 40 % en poids de propane.

9. Utilisation d'un gaz de réaction pour la préparation d'acroléine, **caractérisée en ce que** le gaz de réaction contient 125 ppm en poids ou plus de soufre sous forme de quelconques composants à base de soufre, en chaque cas le gaz de réaction ne contenant pas plus de 5 000 ppm en poids de soufre sous forme de quelconques composants à base de soufre et pas plus de 5 000 ppm en poids d'hydrocarbures insaturés, choisis dans le groupe constitué par C₂H₄, C₃H₄, C₄H₈, C₄H₆, C₅H₁₀, C₅H₈ et des mélanges correspondants.

10. Utilisation selon la revendication 9, le gaz de réaction contenant plus de 50 ppm en poids d'hydrocarbures insaturés, choisis dans le groupe constitué par C₂H₄, C₃H₄, C₄H₈, C₄H₆, C₅H₁₀, C₅H₈ et des mélanges correspondants.
